# EUROPEAN PATENT APPLICATION

(11) **EP 2 420 556 A1**
(43) Date of publication of application: **22.02.2012**
(21) Application number: 11177470.9
(22) Date of filing: 12.08.2011
(51) Int. Cl.: C11C 3/10, C11C 3/08, C12P 7/64

(54) **Replacement fats for cocoa butter having good thermal resistance and mouth-feel and chocolate composition including the same**

(30) Priority: 16.08.2010 KR 20100078722
(71) Applicant: CJ CheilJedang Corporation, Jung-gu Seoul 100-749 (KR)
(72) Inventor: Kim, Chul Jin, 680-764 Nam-gu, Ulsan (KR); Lee, Min Hyun, 151-812 Gwanak-gu, Seoul (KR); Kang, Ji Hyun, 157-773 Gangseo-gu, Seoul (KR); Lee, Yun Jeong, 704-807 Dalseo-gu, Daegu (KR); Lim, Chun Son, 120-795 Seodaemun-gu, Seoul (KR)
(74) Representative: Nevant, Marc

(57) **Abstract**

The present invention relates to a replacement fat for cocoa butter having a POP content of 10 wt% or less based on the total weight of the replacement fat for cocoa butter and a POS/SOS triglyceride content ratio of 1.0 to 1.5. The replacement fat for cocoa butter is prepared by a method including: fractionating a vegetable fat; preparing a raw material fat by mixing the fractionated vegetable fat with a fatty acid derivative; and enzymatically transesterifying the raw material fat.

## Description

### [Technical Field]

The present invention relates to a cocoa butter replacement fat for chocolate and more particularly to a replacement fat for cocoa butter which is prepared by a method including fractionating vegetable fat, preparing a raw material fat by mixing the fractionated vegetable fat with a fatty acid derivative, and enzymatically transesterifying the raw material fat, and has a POP content of 10 wt% or less based on a total weight of the replacement fat for cocoa butter, and triglycerides of POS/SOS content ratio of 1.0 to 1.5. The present invention also relates to a chocolate composition using the replacement fat for cocoa butter.

### [Background Art]

### 1. Cocoa butter

Chocolate generally contains 50% or less of sugar, 30 to 50% of cocoa mass, and about 30% of fat including milk fat. The content of cocoa butter among chocolate fat varies according to the kind of chocolate, but is generally about 60%.

Cocoa butter (cacao butter) is fat obtained from the seed (cacao bean, fat content: 48 to 49 %) of a theobroma cacao fruit. Cocoa butter consists of 98 % of triglyceride, 1 % of free fatty acid, 0.5 % of monoglyceride or diglyceride, 0.2 % of sterol, and 150 to 250 ppm of tocopherol. Triglyceride of cocoa butter has a symmetric structure at 75 % or more in which oleic acid is located in the sn-2 position and palmitic acid and stearic acid are located in the sn-1 and sn-3 positions, respectively. Cocoa butter includes 34 to 49 % of POS, 23 to 30% of SOS, and 13 to 17 % of POP, which mainly form symmetric fat.

Herein, the term "POP" refers to triglyceride in which oleic acid is located in the sn-2 position and palmitic acids are located the sn-1 and sn-3 positions.

The term "POS" refers to triglyceride in which oleic acid is located in the sn-2 position and palmitic acid and stearic acid or stearic acid and palmitic acid are located in the sn-1 and sn-3 positions, respectively.

The term "SOS" refers to triglyceride in which oleic acid is located in the sn-2 position and stearic acids are located in the sn-1 and sn-3 positions.

Unless defined otherwise, the units "%" and "part" denote "% by weight (wt%)" and "part by weight," respectively.

Cocoa butter has a melting point of 32 to 35°C and a solid fat content (SFC) of 71 to 88 % at room temperature (about 20°C), starts melting at 30 to 32°C, and substantially melts at 32 to 35°C.

Since cocoa butter rapidly melts at around 30°C, cocoa butter is solid at room temperature, whereas it quickly melts in the mouth and thus provides refreshing and neat mouth-feel. Such melting characteristics of cocoa butter are due to symmetric fat.

Cocoa butter has different compositions and contents of triglyceride depending on places of origin, which cause differences in properties, such as solidity, solidification speed, or the like. For example, as for compositions of symmetric fat having oleic acid in the sn-2 position, Malaysian cocoa butter has a POS content of 47 % and an SOS content of 30%, while Brazilian cocoa butter has a POS content of 40% and an SOS content of 22 % and Ghanaian cocoa butter has a POS content of 43 % and an SOS content of 26 %. These three cocoa butters have similar POP contents ranging from 13 to 15 %. Regarding solidity, Malaysian cocoa butter is the solidest, Brazilian is the softest, and Ghanaian has a medium level of solidity. Further, solidification speed is different in the same order as solidity, which is 78 + 10 minutes in Malaysian cocoa butter, 300 ± 51 minutes in Brazilian cocoa butter, and 95 ± 14 minutes Ghanaian cocoa butter.

### 2. Replacement fats for cocoa butter

Since cocoa butter is obtained from a natural plant, its supply is changed according to weather changes. Further, since cocoa butter is expensive, vegetable fats are used for chocolate as a substitute for cocoa butter. Such substitute fats include hardened palm kernel oil and coconut oil, which are not compatible with cocoa butter. Substitute fat and oil for cocoa butter is classified into a cocoa butter equivalent and extender (CBE), a cocoa butter replacer (CBR), and a cocoa butter substitute (CBS) depending on production methods and constituents.

The CBE is compatible with cocoa butter, has a similar triglyceride composition to cocoa butter, and needs tempering. Examples of the CBE include a palm middle fraction (PMF), Sal fat, Borneo tallow, Kokum, Shea butter, and fat fractions thereof. It is known that cocoa butter is mixed with a palm middle fraction and fat having a high SOS content to prepare fat similar to cocoa butter.

The CBR is solidified fat obtained by hardening soybean oil, canola oil, palm oil, or the like in a liquid state or in a liquid and solid mixed state at room temperature. The CBR can replace cocoa butter to a certain extent and does not need tempering. The CBR has a steep slope of an SFC curve and high oxidation stability due to increase in melting point and SFC. However, since the CBR is prepared using part hardening, it has a high content of trans acid and thus is nutritionally deficient.

The CBS is obtained by hardening some of vegetable oil and fat, is not compatible with cocoa butter, has a high content of lauric acid, and does not need tempering. The CBS is commonly used for coating in the baking field and is generally prepared by hardening or transesterifying palm kernel oil and coconut oil and, as necessary, mixing with other types of hardened vegetable oil. However, in the presence of moisture, fat and oil having a high content of lauric acid are hydrolyzed due to mold, emit abnormal odor, and have nutritional defect due to lauric acid.

Since the CBR and CBS have nutritional defect and decreased functions associated with texture, e.g., they quickly melt in the mouth, the CBE is increasingly used. CBE is mostly prepared by mixing SOS rich fat synthesized via enzymatic esterification with palm mid-fraction (PMF) obtained by fractionation of palm oil at about 1:1. A CBE generally has a triglyceride composition consisting of 30 to 35 % of POP, 10 to 15 % of POS, and 30 to 35 % of SOS, which has a higher content of POP and SOS and a lower content of POS than the triglyceride composition of Ghanaian cocoa butter (POP: 17 %, POS: 43 % SOS: 26 %).

Physical properties of fat are identified through solid fat content (SFC) at different temperatures. SFC between 20 and 25°C denotes hardness of fat, SFC between 25 and 30°C denotes heat resistance, and SFC at 35°C or more denotes waxiness, which refers to an extent to which fat remains without quickly melting. Cocoa butter or cocoa butter replacement fat used for chocolate is considered to have good quality when their SFC is high at 30°C or less, sharply decreases at 30°C or more, and is very low at 35°C or more, i.e. when SFC forms a steep curve.

Comparing the SFC of cocoa butter with that of the CBE, the CBE having a high SOS content has a lower SFC at 30°C or less than the cocoa butter. However, the CBE having a high SOS content has a higher SFC at 30°C or more than cocoa butter, and thus has a solid feel and leaves a substantial aftertaste in the mouth. As described above, the difference in SFC between the cocoa butter and the CBE, i.e. property difference, is due to the difference in triglyceride composition between the cocoa butter and the CBE. The CBE has a high content of POP and SOS, whereas the cocoa butte has a high POS content. POS and POP have melting points around 35°C, while SOS has a melting point of 41°C. Fat having a high SOS content is relatively hard at 30°C or more (Aleksandra Torbica et al., Eur. Food Res Technol., 2006, 222:385-391).

Recently, there is a trend toward chocolate which is soft rather than hard and quickly melts in the mouth to be neat without leaving an aftertaste. Accordingly, a soft CBE which has decreased SOS content and increased PMF content is developed. The soft CBE has a triglyceride composition including 40 to 45 % of POP, 10 to 15 % of POS, and 30 to 35 % of SOS. The soft CBE has a low SFC overall in a temperature range of 20 to 35°C, and thus it can provide soft-texture chocolate but does not form solid crystals at room temperature to possibly cause a blooming phenomenon.

Most of the currently available CBEs are allowed to have hard or soft properties by adjusting a mixed ratio between SOS obtained through synthesis or fractionation and PMF having a high POP content. However, the CBEs do not exhibit a SFC curve having a steep slope, unlike natural cocoa butter.

US Patent No. 4,705,692 discloses a substitute composition for cocoa butter which includes SOS, POS, and POP and has a high SOS content, in which the ratio of stearic acid to palmitic acid is 1.5:1 1 to 6.0:1.

JP Unexamined Patent Publication No. 1999-243982 discloses fat having a triglyceride composition with a high POS content, produced by transesterification, wherein POS content is low, 18 wt% or less, whereas POP content is high, 10 to 55 wt%, and SOS content is high,10 to 50 wt%. In this respect, the process focuses on synthesizing SOS rather than POS.

JP Unexamined Patent Publication No. 2008-154555 discloses fat and oil for chocolate having proper heat resistance and meltability in the mouth, wherein the fat and oil has an SOS content of 40 to 60 wt%, a POP content of 1 to 10 wt%, and a weight ratio of SOS content/the sum of POS and SOA contents of 1.1 to 1.8, and the SOS content is greater than the sum of the POS and SOA contents. Thus, the fat and oil includes a high SOS content.

These conventional methods focus on fat and oil for chocolate having a high SOS content in order to produce chocolate products which are soft and quickly melt in the mouth and thus do not leave an aftertaste, whereas they do not put emphasis on POS abundant in natural cocoa butter. Further, there has not yet been conducted a study on cocoa butter replacement fat having a high POS content and a controlled POP content with a low melting point among symmetric structures.

In particular, since most of the currently used CBEs have a high POP content and a high SOS content, they feel hard and leave a considerable aftertaste. A soft CBE has crystals which are not solid at room temperature, thereby causing a blooming phenomenon.

Thus, there is a need to develop a new replacement fat that briskly melts in the mouth, has soft texture, exhibits good heat resistance, and forms solid crystals at room temperature so as not to cause a blooming phenomenon.

### [Technical Problems]

The present invention is directed to solving problems that a conventional CBE feels slightly solid and leaves a substantial aftertaste in the mouth and a conventional soft CBE causes a blooming phenomenon at room temperature, and one aspect of the present invention is to provide replacement fat for cocoa butter which briskly melts in the mouth like natural cocoa butter, provides soft texture, has good heat resistance, and does not cause a blooming phenomenon.

Another aspect of the present invention is to provide replacement fat for cocoa butter having excellent texture, quality, functional or preservative properties to improve or maintain the quality of chocolate when it is used for chocolate instead of natural cocoa butter.

A further aspect of the present invention is to provide a chocolate composition or a chocolate composition for coating using the replacement fat for cocoa butter.

### [Technical Solution]

In accordance with an aspect of the present invention, replacement fat for cocoa butter is prepared by enzymatic transesterification, has a POP content of 10 wt% or less based on the total weight of the replacement fat for cocoa butter, and has a POP/SOS triglyceride content ratio of about 1.0 to 1.5, preferably 1.5. It is not easy to obtain a POP/SOS triglyceride content ratio of 1.0 or more while maintaining the POP content to be 10 wt% or less. If the POP content is greater than 10 wt% and the POS/SOS triglyceride content ratio is 1.0 or more, soft texture can be obtained, whereas heat resistance is inappropriate. If the POP content is less than 10 wt% and the POS/SOS triglyceride content ratio is 1.0 or less, texture is not sufficiently soft but is hard. Thus, when the POP content is 10 wt% or less based on the total weight of the replacement fat for cocoa butter and the POS/SOS triglyceride content ratio is about 1.0 to 1.5, good heat resistance and similar texture or characteristics (brisk melting) to natural cocoa butter can be obtained.

Although there is a slight difference in POP content depending on producing areas, cocoa butter generally has a POP content in the range of 13 to 17 wt%. Further, cocoa butter has a different POS/SOS content ratio depending on producing areas, for example, Malaysian cocoa butter has a POS/SOS content ratio of 1.57 (POS: 47 wt%, SOS: 30 wt%); Brazilian cocoa butter, 1.82 (POS: 40 wt%, SOS: 22 wt%); and Ghanaian cocoa butter, 1.65 (POS: 43 wt%, SOS: 26 wt%). In order to provide similar texture and quality while further improving heat resistance when replacement fat for cocoa butter is used for chocolate instead of cocoa butter, it is necessary to have a similar POS/SOS content ratio to cocoa butter and a lower POP content (see results of the heat resistance test in Example 5).

Conventional methods focus only on increasing SOS content to improve texture of chocolate but do not conduct any study on a POS/SOS content ratio as well as control of POP content. Thus, the present invention discloses for the first time that a replacement fat for cocoa butter has good heat resistance and similar texture and characteristics to natural cocoa butter by adjusting POP content to be 10 wt% or less based on the total weight of the replacement fat for cocoa butter and adjusting a POS/SOS triglyceride content ratio to be about 1.0 to 1.5.

The replacement fat for cocoa butter according to the present invention is prepared by a process including fractionating a vegetable fat or oil; preparing a raw material fat by mixing the fractionated vegetable fat or oil with a fatty acid derivative; and enzymatically transesterifying the raw material fat. The preparation process may further include eliminating the fatty acid derivative from the enzymatically transesterified raw material fat. Eliminating the fatty acid derivative from the enzymatically transesterified raw material fat may be conducted at 0.001 to 30 mbar and 100 to 300°C. The fatty acid derivative may be a stearic acid derivative, e.g., stearic acid ethyl ester or stearic acid methyl ester.

The raw material fat may be a mixture of a vegetable fat or oil with a fatty acid derivative at 1:0.5 to 1:10.

The present invention is achieved as follows. Replacement fat for cocoa butter which has adjusted POP content and POS/SOS content ratio is prepared by enzymatically transesterifying a vegetable fat or oil and adjusting a substrate and reaction time during the reaction. Then, the replacement fat is identified to have a similar configuration to natural cocoa butter through analysis of its triglyceride structure. Further, the replacement fat is applied to a chocolate composition, followed by evaluating functional and preservative properties of the composition, thereby proving that the replacement fat for cocoa butter maintains the quality of chocolate instead of cocoa butter.

Examples of vegetable fat or oil may include any vegetable fat or oil generally used in the art, e.g., coconut oil, palm kernel oil, palm oil, canola oil, sun flower oil, soybean oil, cotton seed oil, rice kernel oil, corn oil, olive oil, shea fat, mango kernel fat, Borneo tallow (oil from Shorea stenoptera or Pentadema butyracea), Sal oil (Sherea robusta oil), kokum oil (Garcinia indica oil), without being limited thereto.

In the present invention, a process of fractionating vegetable fat or oil is to obtain a POP containing fat having a content difference in saturated fatty acids and unsaturated fatty acids from vegetable fat or oil raw materials and may be conducted by a method selected from among dry fractionation and solvent fractionation depending on characteristics of vegetable fat or oil raw materials. In solvent fractionation, any solvent, e.g., hexane, acetone, methyl ethyl ketone, ethanol, or the like, may be used as long as it can dissolve raw material fat.

In the present invention, examples of the fatty acid derivative may include palmitic acid ethyl ester, stearic acid ethyl ester, arachidonic acid ethyl ester, and behenic acid ethyl ester; or palmitic acid methyl ester, stearic acid methyl ester, arachidonic acid methyl ester and behenic acid methyl ester. Preferably, stearic acid ethyl ester or stearic acid methyl ester may be used, without being limited thereto. Any fatty acid and any fatty acid derivative generally used in the art may be used.

In the present invention, the enzymatic transesterification may be used to produce symmetric triglyceride including saturated fatty acids in the sn-1,3 positions and unsaturated acid in the sn-2 position, and may be conducted at 30 to 60°C for 1 to 30 hours using an sn-1,3 position-specific enzyme.

Examples of the sn-1,3 positions-specific enzyme may include enzymes extracted from Rhizopus delemar, Mucor miehei, Aspergillus miger, Rhizopus arrhizus, Rhizopus niveus, Mucor javanicus, Rhizopus javenicus, Rhizopus oxyzae, Thermomyces lanuginosus, or the like. Preferably, enzymes extracted from Mucor miehei or Thermomyces lanuginosus may be used, without being limited thereto. Any enzyme specific to the sn-1 and 3 positions used in the art may be used.

In the present invention, the ratio of substrates and reaction time may be adjusted in the enzymatic transesterification to adjust POP content and a POS/SOS content ratio. Further, when the reaction is completed, reactants may be mixed with each other to facilitate use change, thereby adjusting POP content and a POS/SOS content ratio. For example, a composition including 13 % of POP, 37 % of POS, and 23 % of SOS and having a POS/SOS content ratio of 1.6 is mixed with a composition including 6.2 % of POP, 33 % of POS, and 34 % of SOS and having a POS/SOS content ratio of 1 at a weight ratio of 5:5 to produce a composition that includes 9.6 % of POP, 35 % of POS, and 29 % of SOS and has a POS/SOS content ratio of 1.2.

Fat prepared using the above initial raw materials and process has a triglyceride composition including 10 wt% or less of POP and having a POS/SOS ratio of about 1.0 to 1.5. In the enzymatic transesterification, a ratio of substrates and reaction time are adjusted or reactants are mixed, thereby producing a composition having 10 wt% or less of POP and having a POS/SOS ratio of about 1.0 to 1.5.

In the present invention a chocolate composition comprises 1 to 30 wt%, preferably 1 to 15 wt%, of the replacement fat for cocoa butter defined above.

### [Advantageous Effects]

As such, replacement fat for cocoa butter according to the present invention exhibits excellent heat resistance due to a low POP content and has an SFC curve with steep slope specific to cocoa butter due to a high POS content similarly to the triglyceride composition of natural cocoa butter. Thus, the replacement fat briskly melts in the mouth and may be used as cocoa butter equivalents having soft texture. Also, the replacement fat is excellent in improving blooming resistance of chocolate and physical properties as compared with conventional replacement fats for cocoa butter, so that it may be used to enhance the quality of cocoa butter. Further, the replacement fat is added to solid cocoa butter to have a triglyceride composition and physical properties similar to those of cocoa butter having soft texture. In addition, the replacement fat for cocoa butter has an increased POS while keeping POP content low to improve blooming resistance of chocolate and melting feeling, thereby considerably contributing to improvement in product quality.

### [Description of Drawings]

Fig. 1 is a high-performance liquid chromatography (HPLC) graph illustrating a triglyceride composition of a replacement fat for cocoa butter according to the present invention; and
Fig. 2 is a graph illustrating SFC of a replacement fat for cocoa butter according to the present invention and SFC of natural cocoa butter (CB).

### [Mode for Invention]

Hereinafter, the present invention will be described in more detail with reference to the following examples. These examples are provided for illustrative purposes only and are not to be in any way construed as limiting the present invention.

### Example 1: Preparation of replacement fat for cocoa butter and analysis of structure of triglyceride

In Example 1, replacement fat for cocoa butter was prepared as follows. A palm fraction was obtained as a raw material fat through solvent fractionation. 1 kg of palm oil was completely dissolved at 60°C and mixed with 10 kg of acetone. After the container was closed with a stopper, the mixture was stirred so that the oil was thoroughly dissolved in the acetone. The mixed solution was stirred at 0°C and 30 rpm for 3 hours and then crystallized, followed by vacuum filtration, thereby separating into palm stearin in a solid state and palm olein in a liquid state. Here, the palm olein had a yield of 60% or more and an iodine value of 60 or less.

The acetone-unremoved palm stearin obtained through fractionation was completely dissolved at 40°C and mixed with additional acetone. The mixture was stirred at 30°C and 30 rpm and then crystallized, followed by vacuum filtration, thereby separating into a crystallized fraction and a palm middle fraction (PMF). Here, the PMF had a yield of 30%, and the palm fraction contained 55 % of POP and had an iodine value of 40.

The palm fraction was mixed with a stearic derivative at a molar ratio of 1:4 such that the total weight was 2 kg, after which the mixture was subjected to transesterification using immobilized sn-1,3-specific lipase, lipozyme RM IM, obtained from Rhizomucor miehei at 50°C for 8, 12, 15, and 20 hours, thereby synthesizing replacement fats for cocoa butter. Then, ethyl ester present in the synthesized fats was evaporated to produce final replacement fats for cocoa butter.

The kind and content of triglyceride in the fats before/after the enzymatic transesterification were identified using HPLC.

Analysis of triglyceride was conducted using HPLC under conditions listed in Table 1. The triglyceride structure of the fat before and after fractionation was analyzed using reverse-phase high-performance liquid chromatography and an evaporative light scattering detector (ELSD). 30 µl of a specimen and 10 ml of hexane were filtered using a PEFE syringe filter (25 mm, 0.2 µm) and then put in a 2-mm vial, and 20 µl of the specimen was injected using an autosampler. Acetonitrile and hexane/isopropanol were used as a solvent A and a solvent B, respectively, and flow rate was 1 ml/min. Solvent gradient elution (A:B, v:v) was conducted for 70 minutes, which was performed at 80:20 for 45 minutes, at 54:46 up to 60 minutes, and then at 80:20 from 60 minutes to 70 minutes.

**TABLE 1**

| | |
|---|---|
| Equipment | Agilent, 1200 HPLC Chemstation |
| Column | NOVA-pack C18 60 Å 4 µm (3.9 x 150 mm, Waters) |
| Detector | Alltech, Evaporative Light Scattering Detector (ELSD) |
| Sample amount | 20 µl |
| Solvent | Acetonitrile:hexane/isopropyl alcohol |
| | Gradient solvent system |
| Detector gain | 1 |
| Detector oven temperature | 80°C |
| Carrier gas | N₂ (1.5 L/min) |

The triglyceride composition of each fat was identified through HPLC, and results are illustrated in Table 2.

**TABLE 2**

| Reaction time | POP | POS | SOS | POS/SOS |
|---|---|---|---|---|
| 8H | 10.5 | 35.4 | 24.3 | 1.5 |
| 12H | 9.2 | 34.8 | 27.3 | 1.3 |
| 15H | 8.3 | 34.0 | 32.1 | 1.1 |
| 20H | 6.2 | 33.3 | 34.2 | 1.0 |

As shown in Table 2, the replacement fat for cocoa butter obtained through 20-hour reaction has a triglyceride composition including 6.2 % of POP, 33.3 % of POS, and 34.2 % of SOS and has a POS/SOS content ratio of about 1, and the replacement fat for cocoa butter obtained through 12-hour reaction has a triglyceride composition including 9.2 % of POP, 34.8 % of POS, and 27.3 % of SOS and has a POS/SOS content ratio of about 1.3.

### Experimental Example 1: Analysis of SFC using nuclear magnetic resonance (NMR)

In Experimental Example 1, the replacement fat for cocoa butter having a POS/SOS content ratio of 1.3, similar to a POS/SOS content ratio of cocoa butter of 1.57, was selected among the fats obtained in Examples 1 and analyzed as to SFC using nuclear magnetic resonance (NMR), compared with natural cocoa butter. SFC analysis was conducted using NMR under conditions listed in Table 3.

An SFC analysis test using NMR was conducted by a parallel method. Five 3-ml samples were prepared and sufficiently melted at 80°C in pre-treatment, followed by cooling at 60°C for 10 minutes and then at 0°C for 90 minutes. Then, the crystals were stabilized at 26°C for 40 hours and then cooled at 0°C for 90 minutes. The samples were left for 30 minutes in a Celsius bath with a metal block thermostat preset to 10.0°C, 20.0°C, 25.0°C, 30.0°C, and 35.0°C, respectively, followed by SFC measurement. SFC was measured for about 6 seconds.

**TABLE 3**

| | |
|---|---|
| NMR equipment | BRUKER, minispec |
| Frequency | 60 MHz |
| Sample amount | 3 ml |
| Pre-treatment temperature | 100 metal block thermostat, 0°C |
| Experiment temperature | 10.0°C, 20.0°C, 25.0°C, 30.0°C, 35.0°C |

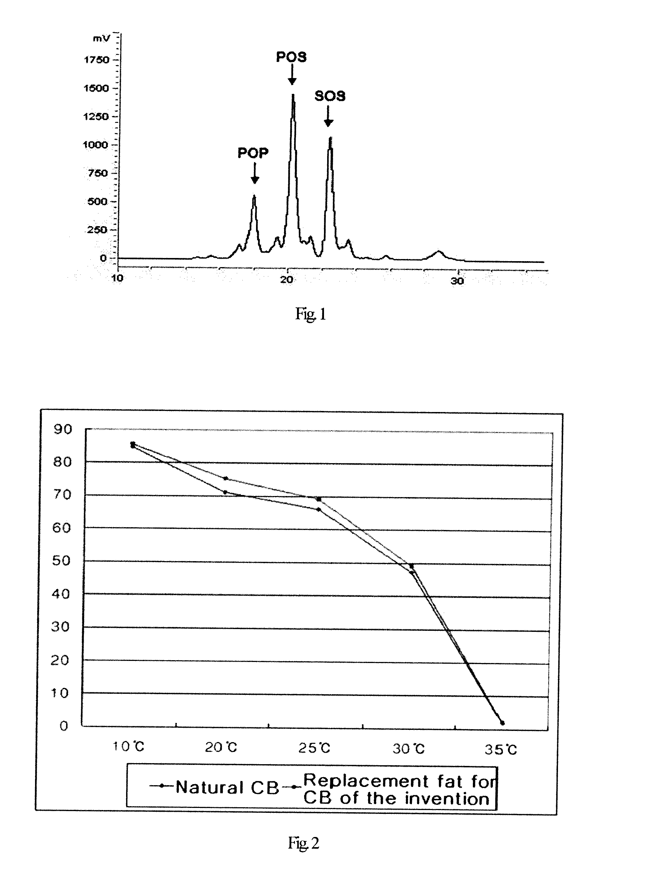

Analysis results of SFC using NMR are illustrated in Fig. 2. As shown in Fig. 2, the selected replacement fat for cocoa butter of the present invention exhibits similar physical properties to natural cocoa butter.

### Example 2: Preparation of chocolate using cocoa butter

In Example 2, chocolate using cocoa butter only as fat was prepared for reference in order to identify whether a chocolate composition using the selected replacement fat for cocoa butter had improved quality, such as mouth-feel and blooming prevention.

A plate-shaped chocolate mixture having a total fat content of 36 % and a particle size of 20 µm was used for reference. To make this chocolate, 27 % of cocoa, 9.6 % of cocoa powder, 20% of cocoa butter, 43 % of sugar, and 0.4 % of lecithin were used. The final composition of the chocolate is listed in Table 4.

**TABLE 4: Composition of plate-shaped chocolate**

| Raw material | Mixing ratio (%) |
|---|---|
| Cocoa mass | 27 |
| Cocoa powder | 9.6 |
| Cocoa butter | 20 |
| Sugar | 43 |
| Lecithin | 0.4 |

First, the sugar, the cocoa powder, and 10% of the mixed fat were mixed into dough, which was subjected to a refiner to have a particle size of 20 µm. The thus obtained flakes were put in a conche and subjected to conching for 20 hours, after which 10% of the remaining mixed fat and the lecithin were added thereto, followed by further conching for 1 hour. The mixture was subjected to tempering from 28°C to 29.5°C, poured into a plate-shaped mold, cooled in a cooling chamber at 10°C for 10 minutes, and taken out of the mold, thereby completing the chocolate.

### Example 3 and Comparative Example 1: Preparation of plate-shaped chocolate using replacement fat for cocoa butter

In Example 3, plate-shaped chocolate was prepared using the replacement fat for cocoa butter containing 9.2 wt% of POP and having a POS/SOS content ratio of 1.3 obtained in Example 1 to identify quality improvement effects, such as improvement in mouth-feel and prevention of blooming, when the cocoa butter used for the chocolate of Example 2 was replaced by the replacement fat. Further, in Comparative Example 1, an experiment of comparing the replacement fat for cocoa butter of Example 1 with commercially used CBE was conducted. The triglyceride composition of the commercially used CBE in the experiment is listed in Table 5.

**TABLE 5: Triglyceride composition of commercially used CBE**

| Composition | POP | POS | SOS |
|---|---|---|---|
| Content (%) | 43.6 | 8.3 | 45.3 |

In Example 2 and Comparative Example 1, chocolate was prepared in the same manner as in Example 2 using 15 % of the 20% cocoa butter replaced by the replacement fat of Example 1 and the commercially used CBE, respectively, and the remaining 5 % of cocoa butter. Examples and Comparative Example are illustrated as follows.

**TABLE 6: Composition of chocolate mixtures according to Examples and Comparative Example**

| Raw material | Example 2 (%) | Example 3 (%) | Comparative Example 1 (%) |
|---|---|---|---|
| Cocoa mass | 27 | 27 | 27 |
| Cocoa powder | 9.6 | 9.6 | 9.6 |
| Cocoa butter | 20 | 5 | 5 |
| Replacement fat | - | 15 | - |
| General CBE | - | - | 15 |
| Sugar | 43 | 43 | 43 |
| Lecithin | 0.4 | 0.4 | 0.4 |

### Example 4: Test of feeling of chocolate melting in mouth

The feeling of the chocolates of Examples 2 and 3 and Comparative Example 1 melting in the mouth was evaluated by 10 participants. 'Good' was indicated by O, 'average' was indicated by Δ, and 'bad' was indicated by X.

**TABLE 7: Feeling of chocolate melting in mouth**

| | Example 2 | Example 3 | Comparative Example 1 |
|---|---|---|---|
| Melting feeling | ○ | ○ | Δ |

| | | | |
|---|---|---|---|
| <Evaluation > ○: Good, Δ: Average, X: Bad | | | |

### Example 5: Heat resistance test of chocolate

The chocolates of Examples 2 and 3 and Comparative Example 1 were aged at 20°C for 1 week, followed by an experiment under the following conditions in order to measure maximum stress by temperature and thus to identify heat resistance.
* Storing time of chocolate in a constant-temperature oven: 3 hours
* Rheometer conditions
Range: 3mm, Table speed: 2 cm/min, Tip: 3-mm-diameter cylindrical tip Measurement results by temperature are illustrated as follows.

**TABLE 8: Maximum stress by temperature**

| Tem perature | | 20°C | 22°C | 24°C | 26°C | 28°C | 30°C | 32°C |
|---|---|---|---|---|---|---|---|---|
| Max Strength (g) | Example 2 | 1100 | 1100 | 1109 | 1108 | 1101 | 390 | 110 |
| | Example 3 | 1205 | 1185 | 1150 | 1132 | 1130 | 532 | 140 |
| | Comparative Example 1 | 1186 | 1180 | 1143 | 1006 | 1000 | 430 | 124 |

As illustrated in Table 8, the chocolate of Example 3 has relative higher maximum stress than the other chocolates, so that it exhibits relatively superior heat resistance.

### Example 6: Blooming stability test of chocolate

The chocolates of Examples 2 and 3 and Comparative Example 1 were aged at 20°C for 1 week and stored in a constant-temperature oven maintained at 32°C for 24 hours and 20°C for 24 hours, followed by a cycling test. This test was conducted 15 times, and then a change in the quality of the chocolates, i.e. occurrence of blooming or graining, was observed with the naked eye over time. Results are illustrated in Table 9. Further, the feeling of the chocolates melting after the test was evaluated in the same manner as in Example 4, and results are illustrated in Table 9.

**TABLE 9: Blooming and graining of chocolate**

| | Example 2 | Example 3 | Comparative Example 1 |
|---|---|---|---|
| Blooming and graining | No occurrence | No occurrence | No occurrence |
| Melting feeling | ○ | ○ | Δ |

| | | | |
|---|---|---|---|
| <Evaluation > ○: Good, Δ: Average, X: Bad | | | |

The chocolates of Examples 2 and 3 and Comparative Example 1 did not exhibit blooming or graining in storage, which is generally associated with heat resistance of fat at a temperature of 30°C or less with respect to tempered chocolate. Further, considering that the feeling of the chocolates melting in the mouth did not change before and after storage, rearrangement of fat crystals did not occur.

### Example 7: Preparation of chocolate for coating using cocoa butter

In Example 7, chocolate for coating using cocoa butter only as fat was prepared for reference in order to identify whether a chocolate composition using the selected replacement fat for cocoa butter had quality improvement effects for coating, such as improvement in mouth-feel and gloss and prevention of cracking and blooming.

A chocolate mixture for coating having a total fat content of 36 % and a particle size of 20 µm was used for reference. To make this chocolate, 5 % of cocoa, 16.6 % of cocoa powder, 35 % of cocoa butter, 43 % of sugar, and 0.4 % of lecithin were used. The final composition of the chocolate is listed in Table 10.

**TABLE 10**

| Raw material | Mixing ratio (%) |
|---|---|
| Cocoa | 5 |
| Cocoa powder | 16.6 |
| Cocoa butter | 35 |
| Sugar | 43 |
| Lecithin | 0.4 |

First, the sugar, the cocoa, the cocoa powder, and 10% of the cocoa butter were mixed into a dough, which was subjected to a refiner to have a particle size of 20 µm. The thus obtained flakes were put in a conche and subjected to conching for 20 hours, after which 10% of the remaining cocoa butter and the lecithin were added thereto, followed by further conching for 1 hour. The mixture was subjected to tempering from 28°C to 29.5°C, applied to a wafer, and cooled in a cooling chamber at 10°C for 10 minutes, thereby producing a final product.

### Example 8 and Comparative Example 2: Preparation of chocolate for coating using replacement fat for cocoa butter

In Example 8, chocolate for coating was prepared using the replacement fat for cocoa butter containing 9.2 wt% of POP and having a POS/SOS content ratio of 1.3 obtained in Example 1 in order to identify quality improvement effects, such as improvement in mouth-feel and gloss and prevention of blooming and cracking, when the cocoa butter used for the chocolate for coating of Example 7 was replaced by the replacement fat. Then, the obtained chocolate for coating was applied to a wafer in the same manner as in Example 7, thereby producing a final product. Further, in Comparative Example 2, an experiment of comparing the replacement fat for cocoa butter of Example 1 with commercially used CBE was conducted. The triglyceride composition of the commercially used CBE used in the experiment was the same as that used in Comparative Example 1.

**TABLE 11: Composition of chocolate mixtures according to Examples and Comparative Example**

| Raw material | Example 7 (%) | Example 8 (%) | Comparative Example 2 (%) |
|---|---|---|---|
| Cocoa | 5 | 5 | 5 |
| Cocoa powder | 16.6 | 16.6 | 16.6 |
| Cocoa butter | 35 | 5 | 5 |
| Replacement fat | - | 30 | - |
| General CBE | - | - | 30 |
| Sugar | 43 | 43 | 43 |
| Lecithin | 0.4 | 0.4 | 0.4 |

### Example 9: Test of feeling of chocolate melting in mouth

The feeling of the wafers coated with the chocolates having different compositions of fats of Examples 7 and 8 and Comparative Example 2 melting in the mouth was evaluated by 10 participants. 'Good' was indicated by ○, 'average' was indicated by Δ, and 'bad' was indicated by X.

**TABLE 12**

| | Example 7 | Example 8 | Comparative Example 2 |
|---|---|---|---|
| Melting feeling | ○ | ○ | Δ |

| | | | |
|---|---|---|---|
| <Evaluation > ○: Good, Δ: Average, X: Bad | | | |

### Example 10: Blooming stability test of chocolate

The chocolate wafers of Examples 7 and 8 and Comparative Example 2 were aged at 20°C for 1 week and stored in a constant-temperature oven maintained at 30°C for 24 hours and 20°C for 24 hours, followed by a cycling test. This test was conducted 15 times, and then change in the quality of the chocolates, i.e. occurrence of blooming or graining, was observed with the naked eye. Results are illustrated in Table 13. Further, the feeling of the chocolates melting after the test was evaluated in the same manner as in Example 9, and results are illustrated in Table 13.

**TABLE 13: Blooming and graining of chocolate**

| | Example 7 | Example 8 | Comparative Example 2 |
|---|---|---|---|
| Blooming and graining | No occurrence | No occurrence | No occurrence |
| Melting feeling | ○ | ○ | Δ |

| | | | |
|---|---|---|---|
| <Evaluation > ○: Good, Δ: Average, X: Bad | | | |

The chocolates of Examples 7 and 8 and Comparative Example 2 did not exhibit blooming or graining in storage, which is generally associated with heat resistance of fat at a temperature of 30°C or less with respect to tempered chocolate. Further, considering that the feeling of the chocolates melting in the mouth did not change before and after storage, rearrangement of fat crystals did not occur.

### Example 11: Cracking and Gloss test

In Example 11, in order to identify whether the chocolate composition using the selected replacement fat for cocoa butter can improve quality required for chocolate for coating, such as gloss and cracking properties, the following test was conducted.

The chocolate wafers of Examples 7 and 8 and Comparative Example 2 were aged at 20°C for 1 week and observed with the naked eye every week for 4 weeks to identify gloss and cracks on the surface of the chocolates. Results are illustrated in Table 14.

**TABLE 14: Gloss and cracks in chocolates**

| | Example 7 | Example 8 | Comparative Example 2 |
|---|---|---|---|
| Initial | Ⓞ/Ⓞ | Ⓞ/Ⓞ | Ⓞ/Ⓞ |
| After 1 week | Ⓞ/Ⓞ | Ⓞ/Ⓞ | Ⓞ/Ⓞ |
| After 2 weeks | Ⓞ/Ⓞ | Ⓞ/Ⓞ | Ⓞ/○ |
| After 3 weeks | ○/○ | ○/Ⓞ | ○/Δ |
| After 4 weeks | ○/○ | ○/○ | ○/Δ |

| | | | |
|---|---|---|---|
| <Evaluation > X: Very bad, Δ: Bad, ○: Good, Ⓞ Very good | | | |

The chocolates of Examples 7 and 8 and Comparative Example 2 had good gloss even after 4 weeks. The chocolate of Examples 7 and 8 were in a very good or good state against cracking, whereas the chocolate of Comparative Example 2 had slight cracks after 3 weeks. Accordingly, the replacement fat for cocoa butter according to the present invention enables chocolate to have good product value and to stably crystallize and thus can be properly used for chocolate coated products instead of natural cocoa butter.

## Claims

1. A replacement fat for cocoa butter being prepared by enzymatic transesterification, replacing cocoa butter, and having a POP content of 10 wt% or less based on the total weight of the replacement fat for cocoa butter and a POS/SOS triglyceride content ratio of 1.0 to 1.5.

2. The replacement fat for cocoa butter according to claim 1, wherein the replacement fat for cocoa butter is prepared by a method comprising fractionating a vegetable fat, preparing a raw material fat by mixing the fractionated vegetable fat with a stearic acid derivative, and enzymatically transesterifying the raw material fat.

3. A chocolate composition comprising 1 to 30 wt% of the replacement fat for cocoa butter according to claim 1 or 2.

4. The chocolate composition according to claim 3, comprising 1 to 15 wt% of the replacement fat for cocoa butter.

5. A method of preparing a replacement fat for cocoa butter having a POP content of 10 wt% or less based on the total weight of the replacement fat for cocoa butter and a POS/SOS triglyceride content ratio of 1.0 to 1.5, comprising:
fractionating a vegetable fat;
preparing a raw material fat by mixing the fractionated vegetable fat with a stearic acid derivative; and
enzymatically transesterifying the raw material fat.

6. The method according to claim 5, wherein the enzymatic transesterification is conducted at 30 to 60°C for 1 to 30 hours.
